(19) Europäisches Patentamt
European Patent Office
Office européen des brevets

(11) Publication number: **0 114 706**
**B1**

(12) **EUROPEAN PATENT SPECIFICATION**

(45) Date of publication of patent specification: **09.09.87**

(51) Int. Cl.$^4$: **C 07 D 205/04,** A 01 N 43/44

(21) Application number: **84200038.2**

(22) Date of filing: **11.01.84**

(54) **2-Acetidinylacetic acid,its preparation,compositions containing it,and method of sterilising the anthers of a plant.**

(30) Priority: **21.01.83 GB 8301702**

(43) Date of publication of application:
**01.08.84 Bulletin 84/31**

(45) Publication of the grant of the patent:
**09.09.87 Bulletin 87/37**

(84) Designated Contracting States:
**AT BE CH DE FR GB IT LI LU NL SE**

(56) References cited:
**EP-A-0 029 265**

**The file contains technical information submitted after the application was filed and not included in this specification**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Orr, Alexander Ferguson**
**6 Amber Close**
**Teynham Nr. Sittingbourne Kent (GB)**

(74) Representative: **Hunter, Keith Roger Ian et al**
**4 York Road**
**London SE1 7NA (GB)**

**EP 0 114 706 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This application relates to 2-azetidinylacetic acid, to its preparation, to compositions containing it, and to a method of producing $F_1$ hybrid seed using it.

To obtain $F_1$ hybrid seeds, which have many advantages over non-hybrid seeds, seed breeders cross-pollinate carefully selected parent plants. In the case of plants which have hermaphroditic flowers and normally self-pollinate, for example small grain cereal plants, self-pollination is prevented and cross-pollination achieved by removing the (male) anthers from each of the flowers by hand, an operation which is extremely time consuming and requires highly-skilled workers. Much research is being carried out into treatments with chemicals which produce male sterility and thereby achieve the same effect.

Thus it is known from Fiziologia Rasteniya *13, 6* (1966) 978—87 that the (naturally occuring) L-azetidine-2-carboxylic acid inhibits pollen germination *in vitro*. However, Applicant has observed that when this compound is applied to plants, it produces little sterilising effect. In addition the compound appears to be rather phytotoxic.

The Applicants have surprisingly found that 2-azetidinylacetic acid does bring about male sterility in plants without unacceptable side-effects such as phytotoxicity.

The present invention also relates to a process for the preparation of the compound according to the invention, characterised in that it is prepared in a way which is known *per se* for analogous compounds. Such processes include, for example, homologation reactions when starting from 2-carboxyazetidine, which compound is available as a natural product (also in an optically active form).

In particular the invention relates to a process for the preparation of a compound according to the invention, characterised in that a compound of general formula

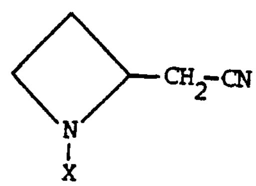

(II)

wherein X represents a hydrogen atom or an alkyl or aralkyl group, preferably having from one to four aliphatic carbon atoms, is hydrolysed followed, when necessary, by replacement of the group X.

The group X is suitably a protective group on the nitrogen atom, e.g. a tosyl, a benzyl or a benzhydryl group, which may be removed by techniques known for de-N-protection of e.g. amino acids, such as hydrogenation or other reductions.

The starting materials for this process, i.e. compounds of formula II as defined hereinabove, are novel, and therefore are included as such within the scope of this invention.

In addition the invention relates to a composition for sterilising the anthers of a plant, comprising the compound according to the invention, together with at least one suitable carrier and/or a surface active agent.

A carrier in a composition according to the invention is any inert material with which the active ingredient is formulated to facilitate application to the plant to be treated, or to facilitate storage, transport or handling. A carrier may be a solid or a liquid, including a material which is normally gaseous but which has been compressed to form a liquid, and any of the carriers normally used in formulating agricultural compositions may be used.

Suitable solid carriers include natural synthetic clays and silicates, for example, natural silicas such as diatomaceous earths; magnesium silicates, for examples, talcs; magnesium aluminium silicates, for example, attapulgites and vermiculites; aluminium silicates, for example, kaolinites, montmorillonites and micas; calcium carbonate; calcium sulfate; ammonium sulphate; synthetic hydrated silicon oxides and synthetic calcium or aluminium silicates; elements, for example, carbon and sulfur; natural and synthetic resins, for example, coumarone resins, polyvinyl chloride, and styrene polymers and copolymers; solid polychlorophenols; bitumen; waxes; and solid fertilizers, for example superphosphates.

Suitable liquid carriers include water; alcohols, for example, isopropanol and glycols; ketones, for example, acetone, methyl ethyl ketone, methyl isobutyl ketone and cyclohexanone; ethers; aromatic or araliphatic hydrocarbons, for example, benzene, toluene and xylene; petroleum fractions, for example, kerosene and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus, preferably, at least one carrier in a composition according to the invention is a surface-active agent. For example, the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulfonic acids; the condensation products of fatty acids or aliphatic amines or

amides containing at least 9 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example, *p*-octylphenol or *p*-octylcresol, with ethylene oxide and/or propylene oxide; sulfates or sulfonates of these condensation product; alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxide.

The compositions of the invention may, for example, be formulated as soluble or wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3—10%w of a dispersing agent and, where necessary, 0—10% w of stabilizer(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilizers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have a thick "mayonnaise"-like consistency.

In many, if not most, cases, the azetidine compound is conveniently applied as a water solution containing a small amount of an inert surfactant, a nonionic material being suitable for the purpose.

Furthermore, the invention relates to a method of sterilizing the anthers of a plant, which comprises applying to the plant a compound or a composition according to the invention.

The method according to the invention generally produces plants in which male sterility has been induced without substantial effect upon the female fertility of the plants. The treated plants thus are quite suitable for use in hybrid seed production. Although, the method of the invention can be used in cases where no fruit set is desired — for example, in cases where a plant is to be used for ornamental foliage only, and it is desirable to avoid the mess caused by unwanted fallen fruit.

This application also relates to a method of producing $F_1$ hybrid seed, which comprises cross-pollinating a plant which has been treated by a method of anther sterilisation according to the invention, with a second plant of a different strain (variety, cultivar, clone), and to the $F_1$ hybrid seed thus produced.

Although the method of the invention is particularly adapted to treatment of cereal grain plants, it is adapted to treatment of flowering plants, generally. The method thus is of interest with respect to the breeding of such crop plants as wheat, barley, oats, rye, flax, hops, maize, sorghum, buckwheat, millet, triticale, sesame, sunflowers, safflower, soybeans, lentils, mustard, cotton, peanuts, rice, rapeseed, sugarbeets, sugarcane and tobacco; vegetables such as tomatoes, beans, peas, celery and onions; grassy and broadleaved forage crops, such as alfalfa, clover, Sudan grass, lespedeza, vetch and grasses; cucurbits such as cucumbers, squash and melons; crucifers (cole crops) such as cabbage, broccoli and cauliflower; and ornamental plants such as annual and perennial plants of interest in the nursery or home garden trades.

The compound or composition may be applied to a plant or its seed at any time during its development, but it appears to have the best effects when it is applied to the plant at a time during the development of the pollen — i.e., between the time of floral initiation and maturation of the pollen, when the pollen grains become independent of the nutritional tissue surrounding them within the anther. Preferably, the azetidine compound is applied somewhat before the pollen is wholly mature, to ensure movement of an effective dosage of the azetidine into the concerned plant tissue, believed to be the pollen grains, in time to effect sterilization of the pollen.

The azetidine is suitably applied at a dosage of from 0.05 to 10 kilograms/hectare, preferably 0.25 to 2.5 kilograms/hectare.

The invention wil be further illustrated by the following examples.

Example 1

Preparation of 2-azetidinylacetic acid.

(a) The methyl ester of DL-(1-benzhydrylazetidin-2-yl)-carboxylic acid (prepared by esterification with methanol of the acid bromide, prepared according to Rodebaugh, R. M. and Cromwell, N. H., Y. Heterocyclic Chem. *6* (1969)435) was dissolved in a quantity of 9 g into 80 ml diethyl ether. This solution was added over 20 minutes to 2 g of $LiAlH_4$ in 50 ml of diethyl ether and the solution was stirred at a reflux temperature for 2 hours. Water (2 ml) was added, then 2 ml 15% aqueous NaOH and finally 6 ml of water. The mixture was stirred for 1 hour and allowed to stand for 15 hours. The solids were filtered off and the filtrate was evaporated to give 8 g of an oily product (1-benzhydryl-2-hydroxymethylazetidine, 99% molar yield).

(b) The product of step (a) (8 g) and 3.2 g triethylamine were mixed in 50 ml toluene, whereupon 3.7 g mesylchloride was added at 0°C over 15 minutes. After stirring for 16 hours at 20°C, the precipitate of triethylammonium chloride was filtered off and the filtrate evaporated. This gave about 11 g of crude product (1-benzhydryl-2-mesyloxymethylazetidine).

(c) The crude product of step (b) was dissolved in 120 ml dimethylformamide and added to a saturated solution of NaCN (4.7 g in 9 ml $H_2O$) at 65°C over 10 minutes. The solution was stirred at this temperature for 16 hours, then cooled and poured onto water. The organic material was extracted with $CH_2Cl_2$, washed with brine, dried and evaporated. The residue was purified by chromatography on silica gel using a solution of ethylacetate (10%) in light petrol as eluent, yielding 5.1 g of product. Recrystallisation from methanol gave 3.9 g of crystalline 1-benzhydryl-2-cyanomethylazetidine, having a melting point of 82—85°C. Elemental analysis (calculated): 82.41% C, 6.92% H, 10.68% N; (found): 82.5% C, 7.0% H, 10.65% N. Mass spectrometry showed a molecular weight of 262 (correct).

(d) The nitrile prepared in step (c) was refluxed with 2.5 g NaOH in a mixture of 36 ml ethanol and 24 ml water for 6 hours and then left to cool for 16 hours. The mixture was poured onto water, washed with ether and acidified to pH 5. No precipitate formed. The solvent was evaporated and the residue was taken up in methanol and filtered to remove the NaCl. The methanol was evaporated and the residue was re-dissolved, in chloroform, washed with brine, dried and evaporated. The residue weighed 5 g, being pure (according to NMR and thin layer chromatography) 1-benzhydryl-2-carboxymethylazetidine (93% molar yield).

(e) The product of step (d) (5 g) and 0.5 g of a 5% palladium on carbon catalyst were shaken in 130 ml methanol at a pressure of 2.8 atm and 50°C. The hydrogen was rapidly taken up. The solution was filtered and evaporated, after which it was partitioned between $H_2O$ and $CHCl_3$. The $H_2O$ layer was washed once with $CHCl_3$ and evaporated. A portion of ethanol was added and evaporated again in order to remove any water, and the residue was warmed under vacuum (1 mm mercury pressure) for desiccation. The resulting crystalline material (2 g) was recrystallised from ethanol to give 1.4 g pure 2-carboxymethylazetidine (formula I wherein $R^1=R^2=R^3=R^4=R^5=R^6=R=H$), having a melting point of 132—137°C (yield 69%). Elemental analysis (calculated) for the hydrate: 45.10% C, 8.33% H, 10.52% N; (found): 45.3% C, 7.1% H, 10.45% N. The mass spectrum showed a molecular weight of 115 (correct). This acid was also converted into its sodium salt (dec. above 200°C).

Example 2

Demonstration of pollen-suppressing activity

Spring wheat, variety SICCO was propagated in a protected environment (i.e. glasshouse or polythene tunnel) in 13 cm pots containing a loam-based compost.

The compound to be tested was formulated as an aqueous solution containing 0.4% Triton (Trade Mark) in 40% acetone being used as the wetter. The formulation was sprayed at a rate of 2 kg/ha in a total spray volume of 650 l/ha.

Male sterility was assessed by placing 3 main stem and 3 tiller ears in cellophane bags from each pot at ear emergence to prevent cross-pollination. At maturity, the bagged ears were harvested, and grain set was recorded, and compared with untreated controls.

The results are shown in the following table.

TABLE 1

| Compound of Example No | Grain Set Inhibition (% of control) | |
|---|---|---|
| | Main Stem | tillers |
| 1 | 85.2 | 74.9 |

It can be seen that the test compound produced a reduction in seed set compared with the untreated controls, clearly illustrating the ability of the compound to induce male sterility in wheat. No phytotoxicity was observed.

**Claims for the Contracting States: BE CH DE FR GB IT LI LU NL SE**

1. 2-Azetidinylacetic acid.

2. Process for the preparation of 2-azetidinyl acetic acid, characterised in that a compound of general formula

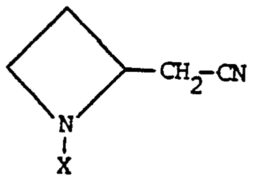

(II)

wherein X represents a hydrogen atom or an alkyl or aralkyl group, claim 1 is hydrolysed, followed, when necessary, by replacement of the group X.

3. Compound according to Formula II as defined in claim 2.

4. Composition for sterilising the anthers of a plant, comprising 2-azetidinylacetic acid, together with at least one suitable carrier and/or a surface active agent.

5. Method of sterilising the anthers of a plant, which comprises applying 2-azetidinylacetic acid to the plant.

**Claims for the Contracting State: AT**

1. Composition for sterilising the anthers of a plant, comprising 2-azetidinylacetic acid, together with at least one suitable carrier and/or a surface active agent.

2. Method of sterilising the anthers of a plant, which comprises applying 2-azetidinylacetic acid to the plant.

3. Process for the preparation of 2-azetidinyl acetic acid, characterised in that a compound of general formula

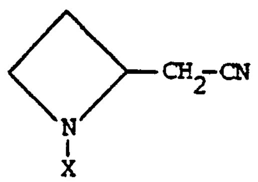

(II)

wherein X represents a hydrogen atom or an alkyl or aralkyl group, claim 1 is hydrolysed, followed, when necessary, by replacement of the group X.

**Patentansprüche für den Vertragsstaaten: BE CH DE FR GB IT LU NL SE**

1. 2-Azetidinylessigsäure.

2. Verfahren zur Herstellung von 2-Azetidinylessigsäure, dadurch gekennzeichnet, daß eine Verbindung der Allgemeinen Formel

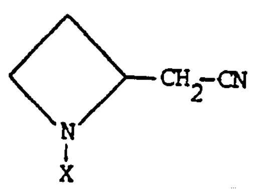

(II)

worin X ein Wasserstoffatom oder eine Alkyl- oder Aralkyl-Gruppe bedeutet, hydrolysiert wird und anschließend, falls erforderlich, die Gruppe X ausgetauscht wird.

3. Verbindung gemäß Formel II, wie in Anspruch 2 definiert.

4. Zusammensetzung zum Sterilisieren der Staubbeutel einer Pflanze, umfassend 2-Azetidylessigsäure zusammen mit wenigstens einem geeigneten Träger und/oder einem oberflächenaktiven Mittel.

5. Verfahren zum Sterilisieren der Staubbeutel einer Pflanze, welches ein Aufbringen von 2-Azetidinylessigsäure auf die Pflanze umfasst.

**Patentansprüche für die Vertragsstaat: AT**

1. Zusammensetzung zum Sterilisieren der Staubbeutel einer Pflanze, umfassend 2-Azetidinylessigsäure zusammen mit wenigstens einem geeigneten Träger und/oder einem oberflächenaktiven Mittel.

2. Verfahren zum Sterilisieren der Staubbeutel einer Pflanze, welches ein Aufbringen von 2-Azetidinylessigsäure auf die Pflanze umfasst.

3. Verfahren zur Herstellung von 2-Azetidinylessigsäure, dadurch gekennzeichnet, daß eine Verbindung der allgemeinen Formel

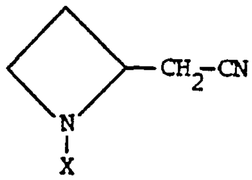

(II)

worin X ein Wasserstoffatom oder eine Alkyl- oder Aralkylgruppe darstellt, hydrolysiert wird, worauf, falls erforderlich, die Gruppe X ausgetauscht wird.

**Revendications pour les Etats contractants: BE CH DE FR GB IT LU NL SE**

1. Acide 2-azétindinylacétique.

2 Procédé de préparation de l'acide 2-azétindinylacétique, caractérisé en ce qu'un composé de formule générale

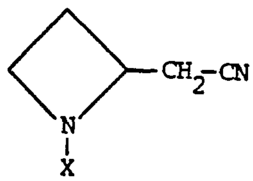

(II)

où X représente un atome d'hydrogène ou un groupe alcoyle ou aralcoyle, revendication 1, est hydrolysé, cela étant suivi, quand c'est nécessaire, du remplacement du groupe X.

3. Composé de formule II tel que défini dans la revendication 2.

4. Composition pour stériliser les anthères d'une plantes, comprenant de l'acide 2-azétidinylacétique en même temps qu'au moins un véhicule et/ou un agent tensioactif appropriés.

5. Méthode de stérilisation des anthères d'une plante, qui comprend l'application d'acide 2-azétidinylacétique à la plante.

**Revendications pour l'Etat contractant: AT**

1. Composition pour stériliser les anthères d'une plante, comprenant de l'acide 2-azétidinylacétique, en même temps qu'au moins un véhicule et/ou un agent tensioactif appropriés.

2. Méthode de stérilisation des anthères d'une plante, qui comprend l'application d'acide 2-azétidinylacétique à la plante.

3. Procédé pour la préparation d'acide 2-azétidinylacétique, caractérisé en ce qu'un composé de formule générale

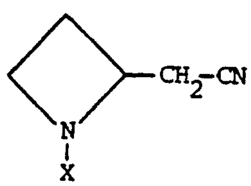

(II)

où X représente un atome d'hydrogène ou un groupe alcoyle ou aralcoyle, revendication 1, est hydrolysé, cela étant suivi, quand, c'est nécessaire, du remplacement du groupe X.